# DEMANDE DE BREVET EUROPEEN

(11) **EP 1 944 380 A1**
(43) Date de publication de la demande: **16.07.2008**
(21) Numéro de dépôt: 08290012.7
(22) Date de dépôt: 08.01.2008
(51) Int. Cl.: C12Q 1/68

(54) **Méthode d'obtention d'un profil génétique spécifique d'une variété d'orge à l'aide de couples d'amorces**

(30) Priorité: 10.01.2007 FR 0700159
(71) Demandeur: Institut Français des Boissons, de la Brasserie et de la Malterie, 54500 Vandoeuvre les Nancy (FR)
(72) Inventeur: Fournier, Régis, 54500 Vandoeuvre les Nancy (FR); Boivin, Patrick, 54850 Messein (FR)
(74) Mandataire: Desaix, Anne

(57) **Abrégé**

La présente invention concerne un ensemble de couples d'amorces, comprenant au moins 5 couples d'amorces, lesdits couples étant choisis dans le but d'obtenir un ensemble de fragments d'ADN spécifique d'une variété végétale d'orge. Cet ensemble est utilisé dans une méthode d'obtention d'un profil génétique d'une variété végétale d'orge et dans une méthode de discrimination de variétés végétales d'orge par comparaison du ou des profil(s) obtenu(s) avec des profils de variétés connues.

SEQUENCE LISTING

<110> INSTITUT FRANCAIS DES BOISSONS, DE LA BRASSERIE ET DE LA
MALTERIE

<120> METHODE D'OBTENTION D'UN PROFIL GENETIQUE SPECIFIQUE D'UNE
VARIETE DORGE A L'AIDE DE COUPLES D'AMORCES

<130> B7068A - AD/LV/KN

<150> FR 07/00159
<151> 2007-01-10

<160> 88

<170> PatentIn version 3.3

<210> 1
<211> 20
<212> DNA
<213> Artificial

<220>
<223> FHVMS455

<400> 1
aagacacaca cacacacgca 20

<210> 2
<211> 20
<212> DNA
<213> Artificial

<220>
<223> RHVMS455

<400> 2
cgaactccat gatgaatccc 20

<210> 3
<211> 20
<212> DNA
<213> Artificial

<220>
<223> FHVMS456

<400> 3
agcttggagt ggaagggaat 20

<210> 4
<211> 20
<212> DNA
<213> Artificial

<220>
<223> RHVMS456

<400> 4
ctgtcagctc ggacaacaaa 20

<210> 5
<211> 20
<212> DNA

<213>  Artificial

<220>
<223>  FHVMS394

<400>  5
accagcaatc caagttacgg                                                    20


<210>  6
<211>  20
<212>  DNA
<213>  Artificial

<220>
<223>  RHVMS394

<400>  6
tgccttggtc ttggtgtgta                                                    20


<210>  7
<211>  20
<212>  DNA
<213>  Artificial

<220>
<223>  FHVMS395

<400>  7
ctcgatatgc atgcttggtg                                                    20


<210>  8
<211>  20
<212>  DNA
<213>  Artificial

<220>
<223>  RHVMS395

<400>  8
cccggctacc aaatgagtaa                                                    20


<210>  9
<211>  20
<212>  DNA
<213>  Artificial

<220>
<223>  FHVMS396

<400>  9
cagagaacca agccaaaacc                                                    20


<210>  10
<211>  20
<212>  DNA
<213>  Artificial

<220>
<223>  RHVMS396

<400>  10
ctatgtttgg tccccttgga                                                    20

<210>  11
<211>  20
<212>  DNA
<213>  Artificial

<220>
<223>  FHVMS397

<400>  11
tccgtacatg ggatgcagta                                                    20


<210>  12
<211>  20
<212>  DNA
<213>  Artificial

<220>
<223>  RHVMS397

<400>  12
cacgtttaac aggaccgaca                                                    20


<210>  13
<211>  20
<212>  DNA
<213>  Artificial

<220>
<223>  FHVMS398

<400>  13
agcgcttcca tccttgacta                                                    20


<210>  14
<211>  20
<212>  DNA
<213>  Artificial

<220>
<223>  RHVMS398

<400>  14
tgactggcac tcacaacaca                                                    20


<210>  15
<211>  20
<212>  DNA
<213>  Artificial

<220>
<223>  FHVMS399

<400>  15
tgtctcccat caaggtttcc                                                    20


<210>  16
<211>  20
<212>  DNA
<213>  Artificial

<220>
<223>  RHVMS399

<400>  16
tcctcttctt tgcgctcttc                                                    20


<210>  17
<211>  20
<212>  DNA
<213>  Artificial

<220>
<223>  FHVMS400

<400>  17
agcttggatg tctggcagtt                                                    20


<210>  18
<211>  20
<212>  DNA
<213>  Artificial

<220>
<223>  RHVMS400

<400>  18
agaccaaatt cacaggccac                                                    20


<210>  19
<211>  20
<212>  DNA
<213>  Artificial

<220>
<223>  FHVMS402

<400>  19
gctaaaccca tcagtggcat                                                    20


<210>  20
<211>  20
<212>  DNA
<213>  Artificial

<220>
<223>  RHVMS402

<400>  20
caaaattgct ctcctcaggg                                                    20


<210>  21
<211>  20
<212>  DNA
<213>  Artificial

<220>
<223>  FHVMS404

<400>  21
ttcacctggc tgctcatgta                                                    20


<210>  22
<211>  20
<212>  DNA

<213>  Artificial

<220>
<223>  RHVMS404

<400>  22
aacttggaat gctgacccag                                                    20


<210>  23
<211>  20
<212>  DNA
<213>  Artificial

<220>
<223>  FHVMS405

<400>  23
tcgagcaggt caagaaggat                                                    20


<210>  24
<211>  20
<212>  DNA
<213>  Artificial

<220>
<223>  RHVMS405

<400>  24
cacggagaca tgcataaacg                                                    20


<210>  25
<211>  20
<212>  DNA
<213>  Artificial

<220>
<223>  FHVMS406

<400>  25
ttttctggtg tgtgtgaggc                                                    20


<210>  26
<211>  20
<212>  DNA
<213>  Artificial

<220>
<223>  RHVMS406

<400>  26
tgtcaagaac ctgctgttgc                                                    20


<210>  27
<211>  20
<212>  DNA
<213>  Artificial

<220>
<223>  FHVMS407

<400>  27
ggaggaaaat aggcacggat                                                    20

<210>  28
<211>  20
<212>  DNA
<213>  Artificial

<220>
<223>  RHVMS407

<400>  28
ggcatctgct cttgatgaca                                                    20


<210>  29
<211>  20
<212>  DNA
<213>  Artificial

<220>
<223>  FHVMS408

<400>  29
gattcgctga tcgaccactt                                                    20


<210>  30
<211>  20
<212>  DNA
<213>  Artificial

<220>
<223>  RHVMS408

<400>  30
gattcgctga tcgaccactt                                                    20


<210>  31
<211>  20
<212>  DNA
<213>  Artificial

<220>
<223>  FHVMS410

<400>  31
gattcgctga tcgaccactt                                                    20


<210>  32
<211>  20
<212>  DNA
<213>  Artificial

<220>
<223>  RHVMS410

<400>  32
atcgatacaa cagtgtgccg                                                    20


<210>  33
<211>  20
<212>  DNA
<213>  Artificial

<220>
<223>  FHVMS412

<400>  33
ggttgccaga aggatgtgat                                                    20

<210>  34
<211>  20
<212>  DNA
<213>  Artificial

<220>
<223>  RHVMS412

<400>  34
tcgcataggc agatacacca                                                    20

<210>  35
<211>  20
<212>  DNA
<213>  Artificial

<220>
<223>  FHVMS413

<400>  35
atgaccagaa aacgcctgtc                                                    20

<210>  36
<211>  20
<212>  DNA
<213>  Artificial

<220>
<223>  RHVMS413

<400>  36
ggattctgca cacacgagaa                                                    20

<210>  37
<211>  20
<212>  DNA
<213>  Artificial

<220>
<223>  FHVMS414

<400>  37
tgtctttagg aacggaggga                                                    20

<210>  38
<211>  20
<212>  DNA
<213>  Artificial

<220>
<223>  RHVMS414

<400>  38
caacacccag atgacgtttg                                                    20

<210>  39
<211>  20
<212>  DNA

<213>  Artificial

<220>
<223>  FHVMS415

<400>  39
cctgttctcc atgtttggct                                                    20


<210>  40
<211>  20
<212>  DNA
<213>  Artificial

<220>
<223>  RHVMS415

<400>  40
atcagtccgg aaagacatgc                                                    20


<210>  41
<211>  20
<212>  DNA
<213>  Artificial

<220>
<223>  FHVMS416

<400>  41
gattcgctga tcgaccactt                                                    20


<210>  42
<211>  20
<212>  DNA
<213>  Artificial

<220>
<223>  RHVMS416

<400>  42
atcgatacaa cagtgtgccg                                                    20


<210>  43
<211>  20
<212>  DNA
<213>  Artificial

<220>
<223>  FHVMS418

<400>  43
tgccatctca agaacacgag                                                    20


<210>  44
<211>  20
<212>  DNA
<213>  Artificial

<220>
<223>  RHVMS418

<400>  44
aagagaactt ccgctccaca                                                    20

<210>  45
<211>  20
<212>  DNA
<213>  Artificial

<220>
<223>  FHVMS419

<400>  45
caactcattc gtcggtgatg                                                    20


<210>  46
<211>  20
<212>  DNA
<213>  Artificial

<220>
<223>  RHVMS419

<400>  46
ggggttggtg acaactcaat                                                    20


<210>  47
<211>  20
<212>  DNA
<213>  Artificial

<220>
<223>  FHVMS420

<400>  47
ttcttgtgtg atactggccg                                                    20


<210>  48
<211>  20
<212>  DNA
<213>  Artificial

<220>
<223>  RHVMS420

<400>  48
cttaaaaccc cgatgaggct                                                    20


<210>  49
<211>  20
<212>  DNA
<213>  Artificial

<220>
<223>  FHVMS422

<400>  49
tgaaggagat caagaacggg                                                    20


<210>  50
<211>  20
<212>  DNA
<213>  Artificial

<220>
<223>  RHVMS422

<400>  50
atcagtccgg aaagacatgc                                                    20


<210>  51
<211>  20
<212>  DNA
<213>  Artificial

<220>
<223>  FHVMS423

<400>  51
tgaaggagat caagaacggc                                                    20


<210>  52
<211>  20
<212>  DNA
<213>  Artificial

<220>
<223>  RHVMS423

<400>  52
atcagtccgg aaagacatgc                                                    20


<210>  53
<211>  20
<212>  DNA
<213>  Artificial

<220>
<223>  FHVMS427

<400>  53
gacatgttgg aaggtggctt                                                    20


<210>  54
<211>  20
<212>  DNA
<213>  Artificial

<220>
<223>  RHVMS427

<400>  54
ctgtcagctc ggacaacaaa                                                    20


<210>  55
<211>  20
<212>  DNA
<213>  Artificial

<220>
<223>  FHVMS429

<400>  55
tgctatcaca ccaatcaggg                                                    20


<210>  56
<211>  20
<212>  DNA

<213>  Artificial

<220>
<223>  RHVMS429

<400>  56
tggacatagt cctggcaaca                                                    20


<210>  57
<211>  20
<212>  DNA
<213>  Artificial

<220>
<223>  FHVMS430

<400>  57
accatcccca cgtaggattt                                                    20


<210>  58
<211>  20
<212>  DNA
<213>  Artificial

<220>
<223>  RHVMS430

<400>  58
tttttcctcc tcctcgtgtg                                                    20


<210>  59
<211>  20
<212>  DNA
<213>  Artificial

<220>
<223>  FHVMS431

<400>  59
cacaccgcac acaacataca                                                    20


<210>  60
<211>  20
<212>  DNA
<213>  Artificial

<220>
<223>  RHVMS431

<400>  60
ttcgttgcat agtggacagc                                                    20


<210>  61
<211>  20
<212>  DNA
<213>  Artificial

<220>
<223>  FHVMS435

<400>  61
cagttggctt ctaccccaaa                                                    20

<210>  62
<211>  20
<212>  DNA
<213>  Artificial

<220>
<223>  RHVMS435

<400>  62
gctacgaccc acaacaacaa                                                    20


<210>  63
<211>  20
<212>  DNA
<213>  Artificial

<220>
<223>  FHVMS436

<400>  63
agtgtgcttg aggccaagat                                                    20


<210>  64
<211>  20
<212>  DNA
<213>  Artificial

<220>
<223>  RHVMS436

<400>  64
gaggattcgc tcaaagttgc                                                    20


<210>  65
<211>  20
<212>  DNA
<213>  Artificial

<220>
<223>  FHVMS475

<400>  65
tctgcacagc tgtttggttc                                                    20


<210>  66
<211>  21
<212>  DNA
<213>  Artificial

<220>
<223>  RHVMS475

<400>  66
caagcagatg caactacacc a                                                  21


<210>  67
<211>  20
<212>  DNA
<213>  Artificial

<220>
<223>  Universal polynucleotide

<400>  67
ccaggacgtt gtaaaacgac                                                    20

<210>  68
<211>  39
<212>  DNA
<213>  Artificial

<220>
<223>  FHVMS394 linked to the universal polynucleotide

<400>  68
cacgacgttg taaaacgaca ccagcaatcc aagttacgg                               39

<210>  69
<211>  39
<212>  DNA
<213>  Artificial

<220>
<223>  FHVMS420 linked to the universal polynucleotide

<400>  69
cacgacgttg taaaacgact tcttgtgtga tactggccg                               39

<210>  70
<211>  39
<212>  DNA
<213>  Artificial

<220>
<223>  RHVMS398 linked to the universal polynucleotide

<400>  70
cacgacgttg taaaacgaca gcgcttccat ccttgacta                               39

<210>  71
<211>  39
<212>  DNA
<213>  Artificial

<220>
<223>  FHVMS404 linked to the universal polynucleotide

<400>  71
cacgacgttg taaaacgact tcacctggct gctcatgta                               39

<210>  72
<211>  39
<212>  DNA
<213>  Artificial

<220>
<223>  FHVMS456 linked to the universal polynucleotide

<400>  72
cacgacgttg taaaacgaca gcttggagtg gaagggaat                               39

<210>  73
<211>  20
<212>  DNA

<213>  Artificial

<220>
<223>  FHVMS401

<400>  73
tgcagatgat gccttctgtc                                                    20


<210>  74
<211>  20
<212>  DNA
<213>  Artificial

<220>
<223>  RHVMS401

<400>  74
tgctgctcaa caaccatagc                                                    20


<210>  75
<211>  20
<212>  DNA
<213>  Artificial

<220>
<223>  FHVMS403

<400>  75
cggacgaggt ttactccaaa                                                    20


<210>  76
<211>  20
<212>  DNA
<213>  Artificial

<220>
<223>  RHVMS403

<400>  76
gctctacgat gcatcccatt                                                    20


<210>  77
<211>  20
<212>  DNA
<213>  Artificial

<220>
<223>  FHVMS409

<400>  77
gataacatcc cggggctaat                                                    20


<210>  78
<211>  20
<212>  DNA
<213>  Artificial

<220>
<223>  RHVMS409

<400>  78
ggatgacatg gctctggttt                                                    20

<210> 79
<211> 20
<212> DNA
<213> Artificial

<220>
<223> FHVMS421

<400> 79
tgtgactgct tctgcagctt 20


<210> 80
<211> 20
<212> DNA
<213> Artificial

<220>
<223> RHVMS421

<400> 80
tgtattcgcc cacaccagta 20


<210> 81
<211> 20
<212> DNA
<213> Artificial

<220>
<223> FHVMS428

<400> 81
acaaaatgag gtcctcgtgc 20


<210> 82
<211> 20
<212> DNA
<213> Artificial

<220>
<223> RHVMS428

<400> 82
tccacaagtt cttccaggct 20


<210> 83
<211> 20
<212> DNA
<213> Artificial

<220>
<223> FHVMS432

<400> 83
gaggggcaca aggaagaaat 20


<210> 84
<211> 20
<212> DNA
<213> Artificial

<220>
<223> RHVMS432

<400>  84
tgtggcacat ccaggaatag                                                    20


<210>  85
<211>  20
<212>  DNA
<213>  Artificial

<220>
<223>  FHVMS433

<400>  85
ttgccttaac cctggtcatc                                                    20


<210>  86
<211>  20
<212>  DNA
<213>  Artificial

<220>
<223>  RHVMS433

<400>  86
ggaatcgtgt cacgtccttt                                                    20


<210>  87
<211>  20
<212>  DNA
<213>  Artificial

<220>
<223>  FHVMS434

<400>  87
ggtgagggca aaagggtaat                                                    20


<210>  88
<211>  20
<212>  DNA
<213>  Artificial

<220>
<223>  RHVMS434

<400>  88
tcgtcatggc agaagtgtgt                                                    20

## Description

La présente invention concerne un ensemble de couples d'amorces, comprenant au moins 5 couples d'amorces, lesdits couples étant choisis dans le but d'obtenir un ensemble de fragments d'ADN spécifique d'une variété végétale d'orge c'est-à-dire représentatif de son identité variétale. L'invention porte également sur un ensemble de fragments d'ADN amplifiés par un ensemble de couples d'amorces selon l'invention.

L'invention fournit également un kit d'obtention d'un ensemble de fragments d'ADN spécifique d'une variété végétale d'orge comprenant un ensemble de couples d'amorces selon l'invention. Dans un mode de réalisation particulier, ces couples d'amorces sont présentés sous forme d'une composition unique.

L'invention porte encore sur une méthode d'obtention d'un profil génétique spécifique d'une variété végétale d'orge c'est-à-dire représentatif de son identité variétale, comprenant l'amplification simultanée d'au moins 5 loci d'une solution d'acides nucléiques obtenue à partir d'un produit dérivé d'orge, au sein d'un unique mélange réactionnel, par un ensemble de couples d'amorces selon l'invention, conduisant à l'obtention d'un ensemble de fragments d'ADN amplifiés, et la détection de l'ensemble formé par lesdits fragments d'ADN amplifiés et ainsi l'obtention d'un profil génétique. Fait également partie de l'invention, une méthode de discrimination de variétés végétales d'orge comprenant une méthode d'obtention d'un profil génétique selon l'invention et la comparaison du ou des profil(s) obtenu(s) avec des profils de variétés connues.

Du fait du grand nombre de variétés végétales d'orge (*Hordeum vulgare*), il est nécessaire de pouvoir identifier avec certitude la nature de la variété végétale utilisée, d'une part, pour s'assurer de sa qualité et, d'autre part, dans le respect de la réglementation sanitaire et alimentaire.

L'identification de variétés végétales a longtemps reposé sur des techniques telles que la visualisation directe du grain ou la croissance de la plante, ou plus récemment sur l'électrophorèse protéique. Cependant, l'utilisation de ces techniques se trouve limitée par la présence sur le marché européen, et plus particulièrement français, d'un grand nombre de variétés végétales d'orge, dont l'identité génétique est proche.

Il existe donc un réel besoin pour une technique d'identification de variétés végétales d'orge avec un nombre limité d'étapes, rapide et bon marché par rapport aux techniques existantes, sans cependant réduire la certitude des résultats. La présente invention se propose de résoudre ces problèmes par l'utilisation d'un faible nombre de couples d'amorces permettant l'obtention d'un profil génétique spécifique, et ainsi de discriminer au moins 50 variétés végétales d'orge.

La présente invention concerne un ensemble de couples d'amorces, dans lequel au moins 5 couples d'amorces, constitués chacun d'une amorce sens et d'une amorce antisens, sont choisis parmi les couples illustrés dans le Tableau 1 ci-dessous. Par conséquent, l'ensemble de couples d'amorces selon l'invention peut comprendre plus de 5 couples, dont au moins 5 couples sont choisis parmi ceux du Tableau 1. Il est ainsi envisageable d'utiliser un ensemble de couples d'amorces qui comprend ou consiste en 6, 7 ou 8 couples choisis parmi ceux du Tableau 1. L'expression « *constitués d'une amorce sens et d'une amorce antisens »* signifie que chaque couple (désigné 1 à 33) identifié dans le Tableau 1 correspond à une seule paire d'amorces à savoir la paire SEQ ID NO:1 / SEQ ID NO:2 pour le couple 1, la paire SEQ ID NO:3 / SEQ ID NO:4 pour le couple 2, et ainsi de suite. Il n'est pas envisageable de mélanger les amorces de différents couples. En effet, chaque couple permet l'amplification d'un locus déterminé. Ainsi, chaque couple d'amorces du tableau 1 permet l'amplification d'un locus différent.

**Tableau 1 : Couples d'amorces**

| **Couple** | **Amorce sens** | | | **Amorces antisens** | | | **Taille moyenne (pb)** |
|---|---|---|---|---|---|---|---|
| | **Nom** | **Séquence (de 5' vers 3')** | **SEQ ID NO :** | **Nom** | **Séquence (de 5' vers 3')** | **SEQ ID NO :** | |
| 1 | **FHVMS455** | AAGACACACACACACACGCA | 1 | **RHVMS455** | CGAACTCCATGATGAATCCC | 2 | 105 |
| 2 | **FHVMS456** | AGCTTGGAGTGGAAGGGAAT | 3 | **RHVMS456** | CTCCACCGTTCTTCACGTTT | 4 | 122 |
| 3 | **FHVMS394** | ACCAGCAATCCAAGTTACGG | 5 | **RHVMS394** | TGCCTTGGTCTTGGTGTGTA | 6 | 124 |
| 4 | **FHVMS395** | CTCGATATGCATGCTTGGTG | 7 | **RHVMS395** | CCCGGCTACCAAATGAGTAA | 8 | 125 |
| 5 | **FHVMS396** | CAGAGAACCAAGCCAAAACC | 9 | **RHVMS396** | CTATGTTTGGTCCCCTTGGA | 10 | 129 |
| 6 | **FHVMS397** | TCCGTACATGGGATGCAGTA | 11 | **RHVMS397** | CACGTTTAACAGGACCGACA | 12 | 134 |
| 7 | **FHVMS398** | AGCGCTTCCATCCTTGACTA | 13 | **RHVMS398** | TGACTGGCACTCACAACACA | 14 | 139 |
| 8 | **FHVMS399** | TGTCTCCCATCAAGGTTTCC | 15 | **RHVMS399** | TCCTCTTCTTTGCGCTCTTC | 16 | 148 |
| 9 | **FHVMS400** | AGCTTGGATGTCTGGCAGTT | 17 | **RHVMS400** | AGACCAAATTCACAGGCCAC | 18 | 155 |
| 10 | **FHVMS402** | GCTAAACCCATCAGTGGCAT | 19 | **RHVMS402** | CAAAATTGCTCTCCTCAGGG | 20 | 170 |
| 11 | **FHVMS404** | TTCACCTGGCTGCTCATGTA | 21 | **RHVMS404** | AACTTGGAATGCTGACCCAG | 22 | 179 |
| 12 | **FHVMS405** | TCGAGCAGGTCAAGAAGGAT | 23 | **RHVMS405** | CACGGAGACATGCATAAACG | 24 | 191 |
| 13 | **FHVMS406** | TTTTCTGGTGTGTGTGAGGC | 25 | **RHVMS406** | TGTCAAGAACCTGCTGTTGC | 26 | 200 |
| 14 | **FHVMS407** | GGAGGAAAATAGGCACGGAT | 27 | **RHVMS407** | GGCATCTGCTCTTGATGACA | 28 | 214 |
| 15 | **FHVMS408** | GATTCGCTGATCGACCACTT | 29 | **RHVMS408** | ATCGATACAACAGTGTGCCG | 30 | 215 |
| 16 | **FHVMS410** | GATTCGCTGATCGACCACTT | 31 | **RHVMS410** | ATCGATACAACAGTGTGCCG | 32 | 215 |
| 17 | **FHVMS412** | GGTTGCCAGAAGGATGTGAT | 33 | **RHVMS412** | TCGCATAGGCAGATACACCA | 34 | 220 |
| 18 | **FHVMS413** | ATGACCAGAAAACGCCTGTC | 35 | **RHVMS413** | GGATTCTGCACACACGAGAA | 36 | 223 |
| 19 | **FHVMS414** | TGTCTTTAGGAACGGAGGGA | 37 | **RHVMS414** | CAACACCCAGATGACGTTTG | 38 | 226 |
| 20 | **FHVMS415** | CCTGTTCTCCATGTTTGGCT | 39 | **RHVMS415** | ATCAGTCCGGAAAGACATGC | 40 | 232 |
| 21 | **FHVMS416** | GATTCGCTGATCGACCACTT | 41 | **RHVMS416** | ATCGATACAACAGTGTGCCG | 42 | 238 |
| 22 | **FHVMS418** | TGCCATCTCAAGAACACGAG | 43 | **RHVMS418** | AAGAGAACTTCCGCTCCACA | 44 | 242 |
| 23 | **FHVMS419** | CAACTCATTCGTCGGTGATG | 45 | **RHVMS419** | GGGGTTGGTGACAACTCAAT | 46 | 247 |
| 24 | **FHVMS420** | TTCTTGTGTGATACTGGCCG | 47 | **RHVMS420** | CTTAAAACCCCGATGAGGCT | 48 | 249 |
| 25 | **FHVMS422** | TGAAGGAGATCAAGAACGGG | 49 | **RHVMS422** | ATCAGTCCGGAAAGACATGC | 50 | 260 |
| 26 | **FHVMS423** | TGAAGGAGATCAAGAACGGC | 51 | **RHVMS423** | ATCAGTCCGGAAAGACATGC | 52 | 260 |
| 27 | **FHVMS427** | GACATGTTGGAAGGTGGCTT | 53 | **RHVMS427** | CTGTCAGCTCGGACAACAAA | 54 | 261 |
| 28 | **FHVMS429** | TGCTATCACACCAATCAGGG | 55 | **RHVMS429** | TGGACATAGTCCTGGCAACA | 56 | 263 |
| 29 | **FHVMS430** | ACCATCCCCACGTAGGATTT | 57 | **RHVMS430** | TTTTTCCTCCTCCTCGTGTG | 58 | 265 |
| 30 | **FHVMS431** | CACACCGCACACAACATACA | 59 | **RHVMS431** | TTCGTTGCATAGTGGACAGC | 60 | 277 |
| 31 | **FHVMS435** | CAGTTGGCTTCTACCCCAAA | 61 | **RHVMS435** | GCTACGACCCACAACAACAA | 62 | 294 |
| 32 | **FHVMS436** | AGTGTGCTTGAGGCCAAGAT | 63 | **RHVMS436** | GAGGATTCGCTCAAAGTTGC | 64 | 295 |
| 33 | **FHVMS475** | TCTGCACAGCTGTTTGGTTC | 65 | **RHVMS475** | CAAGCAGATGCAACTACACCA | 66 | 268 |

Dans la présente invention, les définitions suivantes sont utilisées : Par le terme « *locus »* (au pluriel, des loci), on entend la position de la région amplifiée par le couple d'amorces dans le génome de l'orge. A un locus donné correspondent des *« allèles* », c'est-à-dire des formes différentes de l'ADN du même locus, en terme de composition nucléotidique (remplacement d'une ou plusieurs bases par d'autres) ou en terme de taille (insertion ou délétion d'une ou plusieurs paires de bases, nombre variable de répétions contenues au locus). Un locus est dit *« polymorphe »* lorsque qu'il présente au moins deux allèles observés entre plusieurs génomes, et *« monomorphe »* lorsqu'il présente un seul type d'allèle. L'amplification d'un locus donné permet d'obtenir des fragments d'amplification (ou amplicons ou fragments d'ADN amplifiés), représentatifs des différents allèles présents au locus.

Dans un mode de réalisation particulier, les couples d'amorces selon l'invention correspondent à des loci hautement polymorphes chez l'orge c'est-à-dire que l'amplification d'un génome d'orge produit des fragments amplifiés présentant des tailles variables, selon la variété ou le groupe de variétés. Dans un autre mode de réalisation préféré, les loci amplifiés sont des microsatellites, c'est à dire que chaque locus présente des répétitions dont le nombre est variable d'une variété végétale à l'autre. Ainsi, les couples d'amorces choisis selon l'invention amplifient un locus présentant un nombre d'allèles par locus supérieur à 3. Le nombre d'allèles par locus est préférentiellement compris entre 3 et 10.

Dans un mode de réalisation particulier, le nombre d'allèles par locus est déterminé sur au moins 10, 20, 30, 40, 50, 60 ou 70 ou variétés végétales. Ainsi, un locus polymorphe selon la définition donnée ci-dessus convient aux fins de la présente invention lorsqu'il a été identifié au moins 3 allèles après amplification d'au moins 10 variétés végétales testées. Dans un autre mode de réalisation particulier, le nombre d'allèles attaché à un couple d'amorces de l'invention est supérieur ou égal à 3, 4, 5, 6, 7 ou 8.

L'orge (*Hordeum vulgare*) est une espèce diploïde Ainsi, l'amplification d'un locus donne 2 fragments d'ADN amplifiés (provenant des deux chromosomes d'une même paire) dont la taille peut être identique ou différente. Dans une variété pure, les deux chromosomes d'une paire considérée sont identiques ; après amplification d'un locus, on obtient donc deux fragments de même taille. En revanche, dans une variété hybride, les deux chromosomes d'une paire considérée peuvent être différents ; on obtient donc soit des fragments de taille différente, soit des fragments de taille identique (lignées parentes présentant un allèle de même taille).

Dans un mode de réalisation particulier, les couples d'amorces de l'ensemble selon l'invention sont choisis pour amplifier des loci qui ne sont pas liés génétiquement, c'est-à-dire que la ségrégation d'un locus est indépendante de celle d'un autre locus. Ceci est atteint en choisissant a) des loci présents sur différents chromosomes ou b) des loci présents sur un même chromosome mais à une distance génétique suffisamment élevée pour que la transmission des loci lors d'un phénomène de méiose soit indépendante.

Les couples d'amorces de l'ensemble de l'invention sont choisis afin d'obtenir avec ces seuls couples d'amorces un ensemble de fragments qui s'avère spécifique pour chaque variété végétale d'orge. Par *« spécifique* », on entend que chaque variété végétale d'orge donne, après amplification, avec un ensemble de couples d'amorces selon l'invention un ensemble unique de fragments d'ADN qui est caractéristique de ladite variété et qui peut donc permettre de l'identifier parmi d'autres. En d'autres termes, cet ensemble de fragments d'ADN ainsi obtenu n'est retrouvé chez aucune autre variété végétale d'orge d'un groupe de variétés choisies. En d'autres termes, une variété végétale d'orge peut être caractérisée par un ensemble de fragments d'ADN.

A l'heure actuelle, les ensembles de couples d'amorces selon l'invention permettent d'identifier avec certitude une variété végétale d'orge parmi les variétés végétales d'orge connues en France et en Europe, et particulièrement parmi les variétés végétales d'orge citées dans le tableau 2 suivant. Chaque variété végétale citée dans le tableau 2 donne, après amplification conformément aux conditions de l'invention, un ensemble de fragments d'ADN spécifique et unique, permettant ainsi à l'aide d'un nombre minimum de couples d'amorces de discriminer ces variétés entre elles.

Ainsi, un ensemble selon l'invention comprenant ou consistant en 5 couples d'amorces permet d'identifier une variété végétale d'orge parmi au moins 50 variétés végétales connues. Il est bien entendu que l'ensemble de couples d'amorces selon l'invention est capable de discriminer une variété parmi des groupes de variétés végétales moins importants en nombre, c'est-à-dire au moins 20, au moins 30 ou au moins 40 variétés végétales. Dans un mode de réalisation particulier, 5 couples d'amorces choisis dans le tableau 1 sont capables de discriminer une variété végétale d'orge parmi au moins 50 variétés végétales connues, et particulièrement parmi les 69 variétés végétales d'orge du Tableau 2.

**Tableau 2: Variétés végétales pures susceptibles d'être discriminées avec un ensemble de couples d'amorces selon l'invention.**

| ABONDANCE | BOREALE | CERES | INTRO | MAURITIA | PEWTER | SHAKIRA |
|---|---|---|---|---|---|---|
| ALEXIS | CARAFE | CERVOISE | KANGÔO | MENHIR | PLATINE | SIBERIA |
| ARTHURIO | CARAVAN | CHIPIE | KETOS | NAOMIE | PRESTIGE | SUNRISE |
| ASTORIA | CAROLA | CHOPINE | LAGUNE | NECTARIA | PROPICE | TAVERN |
| AZUREL | CHRISTINA | ESTEREL | MACAW | NEVADA | PUBLICAN | TOCADA |
| AZUREL | COLIBRI | EVEREST | MAESTRIA | NFC TIPPLE | QUENCH | VANESSA |
| BARAKA | CORK | FRANZY | MALWINTA | NICKEL | REGALIA | VERTICALE |
| BEATRIX | DIADEM | GAELIC | MARADO | OPTIC | SCARLETT | VOLGA |
| BELGRANO | DOLMEN | HAMIDA | MASCARA | ORELIE | SEBASTIAN | WESTMINSTER |
| BELLINI | CELLAR | HENLEY | MASSILIA | OROSTAR | SEDUCTION | |

Parmi, les ensembles de couples d'amorces selon l'invention, on citera tout particulièrement un ensemble comprenant ou consistant en les couples d'amorces suivants : a) 2, 3, 7, 11 et 24 cités dans le tableau 1 ci-dessus, b) 2, 10, 16, 17 et 31, c) 1, 11, 16, 18 et 32, et d) 2, 7, 11, 22, 24 et 33. Est compris dans l'invention, un ensemble comprenant 5 couples d'amorces selon le Tableau 1 et éventuellement au moins un autre couple répondant aux mêmes exigences d'hétérozygotie. Un tel ensemble permet de discriminer toutes les variétés végétales d'orge, et au moins 50, 60, 70, 80, 90, 100, 200, 300, 400, 500, 1000 ou 2000 variétés végétales d'orge (pures et/ou hybrides) d'un groupe prédéterminé.

Les couples d'amorces sont choisis pour le nombre important d'allèles qu'ils génèrent après amplification de l'ADN d'orge (marqueurs hautement polymorphes). Ainsi, les couples du Tableau 1, lorsqu'ils sont associés dans des ensembles d'au moins 5 couples, sont capables de générer un nombre important de fragments ADN de taille variable. Ainsi, après amplification avec un ensemble de couples d'amorces selon l'invention, il apparaît possible d'obtenir jusqu'à 10000 ensembles de fragments d'ADN théoriques, se différenciant par le nombre de fragments qu'ils contiennent et/ou par la taille des fragments. Il est donc parfaitement envisageable d'utiliser les ensembles de couples d'amorces selon l'invention pour discriminer une variété végétale d'orge parmi au moins 50, 60, 70, 80, 90, 100, 200, 300, 400, 500, 1000 ou 2000 variétés végétales d'orge (pures et/ou hybrides). La possibilité de discriminer autant de variétés végétales d'orge peut être réalisée aussi bien avec des ensembles de couples d'amorces consistant en 5 couples, ou comprenant 5 couples, et de préférence consistant en 6 ou 7 couples d'amorces.

La présente invention englobe également le remplacement d'au moins un des 5 couples choisis parmi le Tableau 1 par un autre couple d'amorces répondant aux mêmes exigences que celles s'appliquant aux couples du Tableau 1, à savoir une forte hétérozygotie et un grand nombre d'allèles. De même, l'ensemble d'amorces selon l'invention comprend, en plus des couples d'amorces choisis dans le Tableau 1, au moins un couple d'amorces permettant l'amplification d'un locus d'orge, et qui répond aux exigences citées ci-dessus.

Sont compris dans l'invention, les variants fonctionnels des amorces divulguées dans le tableau 1, à savoir les amorces dont la séquence est dérivée par modification d'une séquence d'amorce déterminée du tableau 1 mais qui permet l'amplification du même locus. Un variant fonctionnel doit également répondre aux exigences citées dans la présente demande, en termes de température d'hybridation, de cycle d'amplification et/ou d'absence de compétition avec les autres amorces. De tels variants fonctionnels sont par exemple définis par au moins une des caractéristiques suivantes, éventuellement selon n'importe quelle combinaison :
a. le variant fonctionnel consiste en une amorce dont la séquence a subi la délétion ou la substitution d'au moins 1 nucléotide, préférentiellement 1, 2, 3, 4 ou 5 nucléotides, par rapport à la séquence d'une amorce divulguée dans le tableau 1,
b. le variant fonctionnel consiste en une amorce dont la séquence a subi l'addition (à l'extrémité) ou l'insertion (au sein de l'amorce) d'au moins 1 nucléotide, préférentiellement 1, 2, 3, 4 ou 5 nucléotides, par rapport à la séquence d'une amorce divulguée dans le tableau 1 ; lorsqu'au moins un nucléotide est ajouté, il le sera préférentiellement à l'extrémité 5' de l'amorce, pour éviter de réduire l'efficacité de l'amplification,
c. le variant fonctionnel consiste en une amorce dont la séquence est au moins à 70% similaire à la séquence d'une amorce divulguée dans le tableau 1, préférentiellement au moins à 80% ou 90% similaire, encore plus préférentiellement au moins à 95% similaire. La similarité telle que décrite dans la présente invention est calculée sur l'entièreté des deux séquences d'amorces à comparer, et non pas sur la seule partie commune aux séquences. Par exemple, le variant fonctionnel consiste en une amorce dont la taille est 80%, voire 90% celle de l'amorce du tableau 1, et/ou
d. le variant fonctionnel consiste en une amorce qui hybride dans des conditions stringentes avec une amorce divulguée dans le tableau 1 ; des conditions d'hybridation stringentes se réfèrent à une hybridation d'ADN sur filtre dans une solution de 5X SSC, 0,5% SDS et 100 µ/ml d'ADN, de 55 à 65°C pendant 8 heures, puis un rinçage dans une solution de 0,2X SSC et 0,2% SDS de 60 à 65°C pendant 30 minutes.
Dans un mode de réalisation particulier, et quelle que soit la définition donnée du variant fonctionnel d'une amorce, la taille de l'amorce varie entre 15 et 30 nucléotides, préférentiellement entre 18 et 25 nucléotides (non inclus la présence éventuelle d'un polynucléotide universel).

Dans un mode de réalisation préféré, les amorces selon l'invention comprennent, attaché à l'extrémité 5', un polynucléotide dit universel. Le terme *« universel* », dans la présente demande, signifie que ce polynucléotide présente la même séquence nucléotidique (taille et composition) pour toutes les amorces utilisées au sein d'un même ensemble de couples d'amorces selon l'invention. Le polynucléotide universel est choisi pour ne s'apparier ni à l'une quelconque des amorces de l'ensemble de couples d'amorces selon l'invention, ni aux produits d'amplification résultants (fragments d'ADN amplifiés), ce qui dans les deux cas réduirait l'efficacité de l'amplification et altèrerait la composition de l'ensemble de fragments d'ADN obtenu. Un tel polynucléotide possède par exemple la séquence suivante : CCAGGACGTTGTAAAACGAC (Seq ID N° 67).

Le polynucléotide universel peut être attaché en 5' des amorces sens d'un même ensemble, en 5' des amorces antisens d'un même ensemble ou indifféremment en 5' des amorces sens ou antisens. L'attachement en 5' répond à une contrainte lors de l'amplification, puisque l'amplification est réalisée à partir de l'extrémité 3' des amorces ; par conséquent, alors que les amorces s'hybrident à l'acide nucléique à amplifier, le polynucléotide universel se retrouve lors de l'amplification non-hybridé à l'ADN. Dans un mode réalisation préféré, le polynucléotide universel est fixé à l'amorce de façon covalente.

Dans un mode de réalisation particulier, les couples d'amorces sont choisis pour pouvoir être amplifiés au sein d'un même volume réactionnel, c'est-à-dire que plusieurs amplifications avec des couples d'amorces différents sont réalisées au sein d'une même composition.

La présente invention concerne également un ensemble de fragments d'ADN amplifiés par un ensemble de couples d'amorces selon l'invention, et plus particulièrement un ensemble de fragments d'ADN amplifiés spécifique, selon la définition ci-dessus, d'une variété végétale d'orge. Dans un mode de réalisation particulier, l'ensemble de fragments d'ADN amplifiés selon l'invention est spécifique d'une variété végétale parmi au moins 10, au moins 20, au moins 30, au moins 40, au moins 50, au moins 60, au moins 70, au moins 80, au moins 90, au moins 100, au moins 200, au moins 300, au moins 400, au moins 500, au moins 1000 ou au moins 2000 variétés végétales d'orge connues, et particulièrement parmi les 69 variétés végétales d'orge pures du tableau 2. Ainsi, chaque variété végétale d'orge citée dans le Tableau 2 est caractérisée par un ensemble de fragments d'ADN amplifiés qui lui est propre, c'est-à-dire que cet ensemble n'est retrouvé à l'identique chez aucune autre variété végétale du Tableau 2.

Un ensemble de fragments d'ADN amplifiés est considéré comme « *différent »* d'un ou de plusieurs autre(s) ensemble(s) lorsqu'au moins un des paramètres ci-dessous est vérifié :

- un ensemble présente au moins un fragment qui a une taille différente de la taille des fragments des autres ensembles. Ceci résulte de la nature des allèles amplifiés qui sont de préférence des allèles qui possèdent un nombre de répétions nucléotidiques variables ; ainsi, une variété végétale peut présenter a) un fragment de taille particulière qui lui est caractéristique, c'est-à-dire que ce fragment n'est pas retrouvé chez une autre variété du groupe des variétés considérées ou bien b) une combinaison de fragments particulière qui n'est pas retrouvée chez une autre variété du groupe des variétés considérées ;

- le nombre de fragments d'ADN détectés est différent entre les ensembles ; ceci résulte en partie du fait que bien que le nombre de loci amplifiés soit le même d'une variété végétale à l'autre, les variétés d'orge peuvent présenter des allèles identiques ou différents, en terme de taille, entre les 2 chromosomes d'une même paire. Ceci conduit lors de l'étape de détection à la mise en évidence d'un nombre plus ou moins grand de fragments, selon l'identité ou non en terme de taille des fragments. Ainsi pour deux loci, on peut avoir les combinaisons suivantes :
- locus A : 2 fragments de taille identique et locus B : 2 fragments de taille identique ; on détecte donc deux fragments.
- locus A ou locus B : 2 fragments de taille identique et respectivement locus B ou locus A : 2 fragments de taille différente ; on détecte donc trois fragments.
- locus A : 2 fragments de taille différente et locus B : 2 fragments de taille différente ; on détecte donc quatre fragments.

- un ensemble présente, pour une taille particulière une intensité différente (plus ou moins importante) par rapport à l'intensité d'un autre ensemble (pour un fragment de même taille). Ainsi, deux fragments migrant à la même position correspondant à la même taille donneront une intensité double par rapport à une intensité reflétant la présence d'un seul fragment. L'intensité reflète ici la quantité de fragment d'une certaine taille, dépendamment ou indépendamment de sa séquence nucléotidique. On peut en effet envisager l'amplification de deux loci qui donneraient des allèles dont les tailles se chevaucheraient. Peu importe dans ce cas la séquence nucléotidique du fragment dans la mesure où l'intensité est révélatrice d'une différence dans la composition des deux ensembles et reflète ainsi la nature différente des deux variétés végétales.

C'est ainsi que des ensembles de fragments d'ADN amplifiés présentant un de ces paramètres ou une combinaison d'au moins deux de ces paramètres permettent de discriminer à partir d'un faible nombre de loci un grand nombre de variétés végétales dans la mesure où les couples d'amorces sont choisis pour refléter de façon optimale les différences existantes dans le génome des variétés d'orge à tester par rapport au groupe de variétés de référence.

Dans un mode de réalisation préféré, un ensemble de fragments d'ADN amplifiés est tel qu'obtenu par amplification du génome d'un grain d'orge d'une variété donnée, au moyen d'un ensemble comprenant ou consistant en les couples d'amorces suivants : a) 2, 3, 7, 11 et 24 cités dans le tableau 1 ci-dessus, b) 2, 10, 16, 17 et 31, c) 1, 11, 16, 18 et 32, et d) 2, 7, 11, 22, 24 et 33. Par *« génome d'un grain d'orge* », on entend l'ADN d'une cellule végétale d'un grain d'orge. L'expression *« génome d'un grain d'orge »* couvre également tout acide nucléique issu de l'ADN d'un grain d'orge d'une variété donnée, tel que l'ADN génomique, l'ARN, l'ADNc.

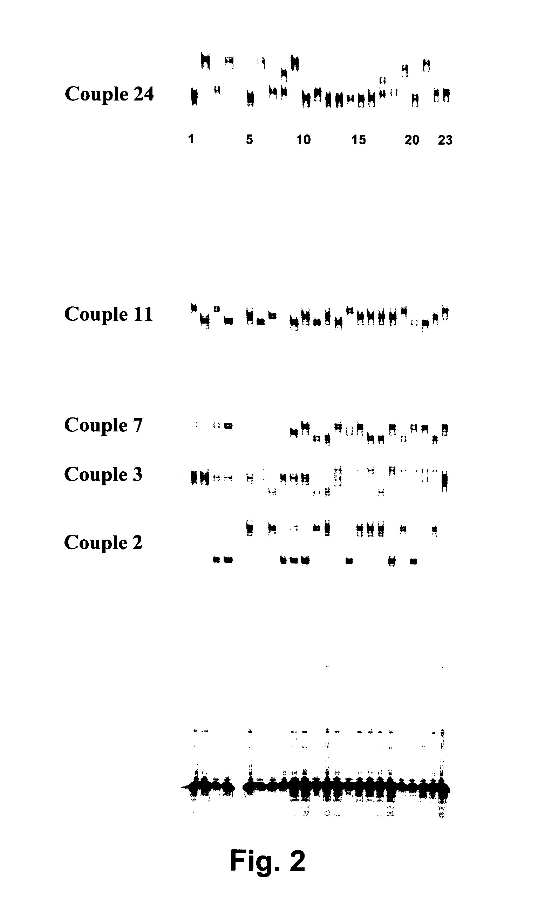

La présente invention concerne également des ensembles de fragments d'ADN ainsi amplifiés qui sont représentés par un profil génétique tel que représenté à la Figure 2. Par *« profil génétique* », on entend toute représentation d'un ensemble de fragments d'ADN amplifiés, permettant de détecter le nombre de fragments, leur taille, leur quantité et/ou leur intensité, au sein de cet ensemble. Alors que l'ensemble de fragments d'ADN amplifiés représente le résultat de l'amplification par les couples d'amorces selon l'invention, le profil génétique représente le résultat de la détection de cet ensemble, par tout moyen approprié, qui finalement permet de distinguer un ensemble de fragments d'ADN amplifiés par rapport à un autre ou d'autres ensemble(s).

Fait également partie de l'invention, un kit d'obtention d'un ensemble de fragments d'ADN amplifiés selon l'invention. Ce kit comprend un ensemble de couples d'amorces selon l'invention, et particulièrement un ensemble de couples d'amorces comprenant au moins 5 couples choisis parmi ceux du Tableau 1 ou leurs variants fonctionnels. Dans un mode de réalisation préféré, un ensemble de couples d'amorces selon l'invention est l'ensemble comprenant ou consistant en les couples d'amorces suivants :
a) 2, 3, 7, 11 et 24 cités dans le tableau 1 ci-dessus,
b) 2, 10, 16, 17 et 31,
c) 1, 11, 16, 18 et 32, et
d) 2, 7, 11, 22, 24 et 33.

Le kit comprend également au moins une solution servant de marqueur de taille, afin d'identifier la taille des fragments amplifiés. Ce marqueur de taille est constitué de fragments dont la taille est connue et qui servent ainsi de référence pour déterminer la taille des fragments d'ADN amplifiés. On peut aussi choisir des marqueurs de taille commerciaux, préférentiellement marqués, tel que le RDye^{™} 700 infrared dye 50-350 sizing standard (Science Tec)... De façon préférée, on choisira comme marqueur de taille un ensemble de fragments d'ADN amplifié à partir de l'ADN d'une variété végétale connue, optionnellement marqué. L'avantage d'un tel marqueur de taille par rapport à un marqueur de taille commercial résulte du fait que certains fragments servant de référence présentent des tailles identiques aux fragments dont la taille est à déterminer, puisque l'origine des fragments est identique, à savoir une variété végétale d'orge. Avantageusement, on utilisera plusieurs ensembles de fragments d'ADN amplifiés comme référence. Dans ce cas, on choisira des ensembles couvrant un large éventail en terme de tailles minimale ou maximale, mais aussi des ensembles possédant des fragments dont l'incrémentation de taille est régulière. De façon avantageuse, on utilisera le même ensemble de couples d'amorce pour l'identification des variétés végétales et pour la réalisation du ou des marqueur(s) de taille. Alternativement, on choisira des mélanges de produits d'amplification provenant de couples et/ou de variétés végétales différents. Ainsi sont préférés, les mélanges suivants :
a) produits d'amplification de l'ADN de la variété Alexis avec le couple 24, de l'ADN de la variété Massilia avec le couple 24, de l'ADN de la variété Mauritia avec le couple 2, de l'ADN de la variété Cervoise avec le couple 7, de l'ADN de la variété Belgrano avec le couple 11, de l'ADN de la variété Chopine avec le couple 33 et de l'ADN de la variété Azurel avec le couple 22 ;
b) produits d'amplification de l'ADN de la variété Mauritia avec le couple 24, de l'ADN de la variété Cervoise avec le couple 2, de l'ADN de la variété Belgrano avec le couples 7, de l'ADN de la variété Chopine avec en multiplex les couples 11 et 22 et de l'ADN de la variété NFC Tipple avec le couple 33 ; et
c) produits d'amplification de l'ADN de la variété Alexis avec le couple 2, de l'ADN de la variété Beatrix avec le couple 7, de l'ADN de la variété NFC Tipple avec en multiplex les couples 11 et 24, de l'ADN de la variété Henley avec le couple 33 et de l'ADN de la variété Sodamm avec le couple 22.

Dans un mode de réalisation préférée, les variétés végétales d'orge choisies pour réaliser ces marqueurs de taille sont au moins une variété végétale d'orge parmi Alexis, Massilia, Mauritia, Beatrix, Cervoise, Belgrano, Chopine, NFC Tipple, Helney, Azurel et Sodamm.

Dans le kit selon l'invention, l'ensemble de couples d'amorces peut se présenter sous la forme de compositions en nombre égal au nombre d'amorces constituant l'ensemble de couples d'amorces ; dans ce cas, l'utilisateur du kit est libre de combiner les différentes amorces dans des concentrations ou rapports entre amorces qu'il souhaite. Dans un autre mode de réalisation, l'ensemble de couples d'amorces peut se présenter sous la forme d'une composition unique, c'est-à-dire que les amorces sont regroupées au sein d'une seule et même composition, la concentration de chaque amorce, et le rapport de chaque amorce par rapport aux autres étant prédéfinis. Les amorces dans la composition de l'invention peuvent se présenter sous forme liquide ou sous forme lyophilisée.

Le kit peut optionnellement comprendre les réactifs nécessaires à l'amplification, et particulièrement à l'amplification en chaîne par polymérase (ACP ou PCR). Ainsi, peuvent faire partie du kit selon l'invention une polymérase, de préférence une polymérase thermostable telle que la *Taq* polymérase, et/ou des desoxyribonucléotide triphosphates (dNTP) dont un peut éventuellement être marqué.

Le kit selon l'invention peut également comprendre un polynucléotide dont la séquence nucléotidique est complémentaire (polynucléotide complémentaire) de celle d'un polynucléotide universel attaché à la partie 5' des amorces de l'invention. Le kit peut également comprendre un manuel divulguant les profils génétiques obtenus pour chaque variété végétale pouvant être testée et/ou les spécificités des profils de ces variétés.

Les amorces selon l'invention ou le polynucléotide complémentaire peuvent éventuellement être marqués de façon radioactive, fluorescente, enzymatique, chemoluminescente.

L'invention se rapporte également à une méthode d'obtention d'un profil génétique spécifique d'une variété végétale. Les termes « *profil génétique »* et *« spécifique »* sont ici utilisés conformément aux définitions données ci-dessus. La méthode de l'invention comprend :
a. l'amplification simultanée d'au moins 5 loci d'une solution d'acides nucléiques obtenue à partir d'un produit dérivé d'orge, au sein d'un unique mélange réactionnel, par un ensemble de couples d'amorces selon l'invention, conduisant à l'obtention d'un ensemble de fragments d'ADN amplifiés, et
b. la détection de l'ensemble formé par lesdits fragments d'ADN amplifiés et l'obtention d'un profil génétique.

Par *"produit dérivé de l'orge",* on entend notamment les matières premières (grain, malt d'orge ou malt spécial), les produits de première transformation (par exemple la farine d'orge), les produits de seconde transformation (alimentation humaine ou animale) et les produits finis (bière, produits contenants de la farine d'orge tels que les biscuits ou les céréales). Par « *malt d'orge* », on entend un grain d'orge germé et séché. Par « *malt spécial* », on entend un grain d'orge séché et/ou torréfié par chauffage à haute température.

Dans un mode de réalisation particulier, la méthode de l'invention est réalisée sur une solution d'acides nucléiques obtenue à partir d'un produit dérivé d'orge, tel qu'un grain d'orge, du malt d'orge, du broyat de grains ou de malt d'orge, de la farine d'orge, de la bière, tout produit de malterie/brasserie, ou tout produit contenant de l'orge, du malt d'orge ou du malt spécial.

Dans un mode de réalisation particulier de l'invention, l'amplification est réalisée par amplification en chaîne par polymérase (ACP ou PCR), bien que toute méthode permettant l'amplification d'un locus à l'aide d'amorces puisse également être utilisée.

L'expression « *amplification simultanée* », signifie que l'amplification des différents loci est réalisée en même temps, c'est-à-dire suivant un programme d'amplification identique quel que soit le locus amplifié. Ainsi, le temps des différentes étapes d'une ACP, à savoir la dénaturation, l'hybridation et l'élongation, sont identiques pour tous les couples d'amorces utilisés.

L'expression « *unique mélange réactionnel »* signifie que les différents couples d'amorces sont utilisés au sein d'un même volume, permettant ainsi l'amplification de plusieurs loci dans une unique procédure d'amplification (communément appelée amplification multiplex). Ainsi, en plus d'être réalisée avec un programme identique en termes de temps de dénaturation, d'hybridation et d'élongation, l'amplification est également réalisée à des températures d'hybridation identiques pour tous les couples d'amorces, puisque les amorces sont contenues dans une unique composition. Plus particulièrement, l'efficacité d'une amplification dépend en grande partie de la stabilité de l'hybridation des amorces avec l'acide nucléique cible, ladite stabilité étant dépendante de la température utilisée pendant l'étape d'hybridation. En effet, c'est la composition nucléotidique des amorces qui détermine la température optimale théorique d'hybridation, celle-ci pouvant cependant se révéler différente dans les conditions expérimentales.

La détermination d'ensembles de couples d'amorces appropriés pour la méthode d'obtention d'un profil génétique spécifique d'une variété végétale a donc fait appel à une sélection, en plusieurs étapes successives, de quelques couples d'amorces parmi un grand nombre de couples d'amorces potentiels :

1^{ère} étape : sélection de couples d'amorces amplifiant des loci polymorphes ; toutes les amorces, que ce soit celles décrites dans l'art antérieur ou celles capables potentiellement d'amplifier un locus de variété végétale d'orge, ne conviennent pas forcément dans la présente méthode (Tableau 7). En effet, un nombre non-négligeable de couples d'amorces amplifient des loci monomorphes (un seul fragment) ou donnent des fragments de taille incorrecte ou une pluralité de fragments réalisant un nuage (smear). Pour la méthode de l'invention, n'ont été retenus que les couples d'amorces amplifiant des loci présentant un fort taux d'hétérozygotie, c'est-à-dire présentant un grand nombre d'allèles, comme expliqué ci-dessus. Le caractère polymorphe d'un locus amplifié par un couple d'amorces déterminé dépend du génome contenant ledit locus, et donc des variétés végétales testées. Il n'est donc pas possible de prévoir, avant la réalisation de l'amplification, quels sont les loci qui seront polymorphes dans les variétés végétales à discriminer. En effet, un locus pourrait être faiblement polymorphe dans un groupe de variétés végétales qui proviendraient d'une lignée commune, mais être fortement polymorphes pour des variétés végétales d'origine différente. Le choix des couples d'amorces ne se fait donc pas en fonction des résultats d'amplification sur une seule variété végétale, mais plutôt en fonction d'un groupe de variétés végétales donné considéré comme groupe de référence.

2^{éme} étape: sélection de couples d'amorces amplifiant à une température d'hybridation expérimentale déterminée ; parmi les couples d'amorces sélectionnées à la première étape, il convient de déterminer ceux qui non seulement sont susceptibles d'amplifier à une température d'hybridation commune, mais aussi sont susceptibles de donner une amplification efficace en terme de qualité (taille de fragments correcte et absence de fragments additionnels qui pourraient résulter de la modification de la température théorique) et en terme de quantité finale de fragments.

3^{ème} étape : sélection de couples d'amorces amplifiant au sein d'un même volume réactionnel. Il est essentiel de préciser à ce stade que la réaction d'amplification requiert une stoechiométrie (proportions des différents réactifs participant à la réaction) assez stricte et n'autorise pas de très grandes variations dans la concentration des différents réactifs (acide nucléique cible, amorces, enzyme, dNTP, sels ..). Ainsi, alors que deux couples d'amorces peuvent donner une amplification d'un loci efficace lorsque les amplifications sont réalisées dans des volumes réactionnels séparés, l'utilisation de ces mêmes couples d'amorces dans un unique volume réactionnel peut conduire à une diminution de l'efficacité de l'amplification d'un des deux loci ou des deux loci.

La réalisation d'une amplification multiplex impose les contraintes suivantes ; il s'agit d'éviter d'une part les phénomènes de compétition entre amorces, c'est-à-dire l'hybridation (même incomplète) de deux amorces à la même séquence nucléique cible, et d'autre part l'hybridation des amorces entre elles ou avec un des produits d'amplification. En effet, toute hybridation d'une amorce à une autre séquence que la séquence cible altère, c'est-à-dire diminue, l'amplification normalement attendue entre les deux amorces du couple, et peut éventuellement conduire à l'amplification de fragments de taille incorrecte.

Les inventeurs ont dans la présente invention identifié des couples d'amorces qui, lorsqu'ils sont utilisés ensemble au sein d'un même volume réactionnel, sont capables d'amplifier efficacement leur locus, et permettent ainsi d'obtenir un ensemble de fragments d'ADN spécifique d'une variété végétale d'orge. Ces couples d'amorces, résumés dans le tableau 1, ont montré, selon plusieurs combinaisons possibles d'au moins 5 couples d'amorces, une amplification efficace d'au moins 5 loci d'une variété végétale d'orge. Un ensemble de couples d'amorces préféré pour la méthode de l'invention est un ensemble comprenant ou consistant en les couples d'amorces suivants : a) 2, 3, 7, 11 et 24 cités dans le tableau 1 ci-dessus, b) 2, 10, 16, 17 et 31, c) 1, 11, 16, 18 et 32, et d) 2, 7, 11, 22, 24 et 33.

Les autres contraintes imposées par une amplification multiplex sont les suivantes :

- il convient de s'assurer qu'au sein d'un même volume réactionnel, chaque réaction d'amplification (c'est à dire chaque réaction d'un couple d'amorces), pourra être correctement réalisée en présence d'une quantité suffisante (non limitative) de réactifs. Par conséquent, les proportions des différents réactifs doivent être adaptées au nombre de couples d'amorces. Ainsi, dans un mode de réalisation préféré, la concentration finale de chaque amorce permettant la réaction d'amplification est comprise entre 10 nM et 1 µM, et particulièrement entre 50 et 500 nM. Dans un autre mode de réalisation préféré, qui peut être dépendant ou indépendant du précédent, la concentration finale d'ADN sur lequel est réalisée l'amplification est comprise entre 10 et 20 ng/µl.

- enfin, on sait qu'il existe une grande variabilité d'efficacité dans la réaction d'amplification entre différents couples d'amorces, provenant probablement, entre autres, de la séquence nucléotidique particulière de la séquence cible à amplifier. Ainsi, dans le cas d'un ensemble de couples d'amorces utilisé dans un seul volume réactionnel, il convient d'adapter la proportion des concentrations des différents couples d'amorces entre eux, afin de favoriser une efficacité aussi identique que possible de toutes les réactions d'amplification. Ainsi, les ensembles de couples d'amorces selon l'invention permettent une amplification efficace aussi bien en terme de qualité qu'en terme de quantité.

L'expression « *détection de l'ensemble formé par lesdits fragments d'ADN amplifiés »* signifie que les fragments d'ADN amplifiés sont identifiés selon des paramètres permettant de distinguer un ensemble de fragments d'ADN amplifiés d'un autre ensemble. De tels paramètres sont par exemple le nombre de fragments, leur taille, leur quantité, leur intensité, leur charge, leur structure tridimensionnelle, ....

Dans un mode de réalisation particulier, la détection des fragments d'ADN amplifiés par l'amplification multiplex requiert leur séparation et leur visualisation. La séparation peut être réalisée selon un unique paramètre, ou bien selon la combinaison d'au moins deux paramètres, par exemple choisis parmi les paramètres suivants : la taille, la charge, la structure tridimensionnelle ....

Dans un exemple de réalisation, les fragments sont séparés selon leur taille, par migration électrophorétique ; les méthodes suivantes bien connues de l'homme du métier peuvent être utilisées afin de séparer les fragments d'ADN amplifiés obtenus dans le cadre de la présente invention : le gel d'électrophorèse (électrophorèse en gel d'agarose ou en gel d'acrylamide) ou les techniques d'électrophorèse capillaire.

A titre d'exemple, la migration éléctrophorétique est effectuée en gel d'acrylamide, et de façon préférée sur un gel hautement résolutif et/ou en conditions dénaturantes (permettant la séparation des deux brins d'ADN d'un fragment amplifié).

La détection (sans séparation) ou la visualisation (après séparation) peut être réalisée par n'importe quelle technique connue, et notamment à l'aide de molécules qui se fixent à l'ADN (traceurs) ou s'intercalent dans l'ADN (agents intercalants) radioactifs, fluorescents, chemoluminescents et enzymatiques. Il est ainsi possible de détecter ou visualiser l'ensemble de fragments à l'aide de bromure d'éthidium. Alternativement, la détection ou la visualisation des fragments d'amplification est réalisée par fluorescence, soit à l'aide d'un fragment nucléotidique marqué par fluorescence ou par l'intermédiaire des amorces sens ou antisens, auxquelles est liée une molécule fluorescente.

Dans un mode de réalisation préféré, la détection ou la visualisation est réalisée à l'aide d'un fragment nucléotidique marqué par fluorescence, qui est complémentaire d'un polynucléotide universel localisé en 5' de l'amorce sens. Par exemple, le fragment marqué par fluorescence est complémentaire de la séquence CCAGGACGTTGTAAAACGAC (Seq ID N° 67). L'utilisation d'un seul fragment nucléotidique marqué (par opposition à une pluralité d'amorces sens ou antisens marquées) permet de réduire les coûts de détection, puisqu'une seule molécule est utilisée pour la détection de l'ensemble des fragments d'ADN amplifiés. Dans un mode de réalisation particulier, le fragment nucléotidique marqué par fluorescence possède, liée en 5', une molécule émettant dans l'infrarouge à 800 nm.

De plus, la méthode d'obtention d'un profil génétique selon l'invention peut optionnellement comprendre des étapes supplémentaires :

(1) une étape d'extraction de l'ADN à partir d'un produit dérivé d'orge (matières premières, produits de transformation ou finis), l'étape d'extraction précédant l'étape d'amplification simultanée multiplex. Dans un mode de réalisation particulier, l'extraction de l'ADN est réalisée par lyse chimique et/ou thermique et/ou enzymatique.

(2) une étape de broyage du grain d'orge, précédant celle d'extraction, par exemple une étape de broyage mécanique, qui peut être réalisée sans addition de tampon.

Afin de rendre la présente méthode d'obtention encore plus rapide, on peut envisager de réaliser l'étape d'amplification, sans purification de l'ADN, c'est à dire directement après une étape optionnelle de broyage et une étape de lyse.

La méthode utilisée ci-dessus répond aux problèmes posés initialement, dans la détection d'une variété végétale d'orge, à savoir la rapidité, le coût, et la certitude des résultats.

Ainsi, l'utilisation d'un ensemble de couples d'amorces au sein d'un unique volume réactionnel accélère la préparation des échantillons pour l'amplification dans la mesure où l'analyse de l'ADN d'une variété végétale requiert une seule réaction d'amplification, et rend sans objet les opérations de mélange des différents produits d'amplification, qui peuvent de plus être source d'erreurs et de contaminations. De plus l'absence de purification de l'ADN après son extraction n'altère pas l'efficacité et la qualité des amplifications, mais réduit de nouveau le temps nécessaire à l'obtention d'un profil génétique spécifique d'une variété végétale. Par conséquent, en réduisant le nombre d'étapes au stade de l'amplification mais également lors des étapes optionnelles antérieures, la méthode est plus rapide et permet d'obtenir un profil génétique environ 8 à 10 heures après le prélèvement du produit dérivé d'orge.

Le coût s'en trouve également diminué du fait de l'utilisation d'un seul volume réactionnel pour l'amplification d'au moins 5 loci ce qui réduit, d'une part, la quantité de réactifs nécessaires et, d'autre part, la consommation de matériel utilisé. Enfin, le coût peut encore être diminué par l'utilisation d'une molécule marquée unique lors de l'étape de détection ou de visualisation.

La présente demande se rapporte également à une méthode de discrimination de variétés végétales de céréales, notamment de variétés d'orge, comprenant une méthode d'obtention d'un profil génétique selon l'invention, et une étape c. de comparaison du ou des profil(s) obtenu(s) avec des profils de variétés connues, notamment les profils de variétés d'un groupe déterminé, considéré comme groupe de référence. Ainsi, la méthode de discrimination comprend :
a. l'amplification simultanée d'au moins 5 loci d'une solution d'acides nucléiques obtenue à partir d'un produit dérivé d'orge, au sein d'un unique mélange réactionnel, par un ensemble de couples d'amorces selon l'invention, conduisant à l'obtention d'un ensemble de fragments d'ADN amplifiés,
b. la détection de l'ensemble formé par lesdits fragments d'ADN amplifiés et l'obtention d'un profil génétique, et
c. la comparaison du ou des profil(s) obtenu(s) avec des profils de variétés connues.

Cette méthode peut comprendre les étapes optionnelles mentionnées ci-dessus pour la méthode d'obtention d'un profil génétique. Toutes les caractéristiques particulières décrites pour la méthode d'obtention d'un profil génétique s'appliquent également à la présente méthode. Dans un mode de réalisation préféré, la méthode de discrimination de variétés végétales de céréales selon l'invention permet de discriminer au moins 10, au moins 20, au moins 30, au moins 40, au moins 50, au moins 60, au moins 70, au moins 80, au moins 90, au moins 100, au moins 200, au moins 300, au moins 400, au moins 500, au moins 1000 ou au moins 2000 variétés végétales d'orge, c'est à dire qu'il est possible de distinguer, avec certitude, une variété végétale d'orge parmi un ensemble de 69 variétés végétales d'orge connues (pures et/ou hybrides), et plus particulièrement parmi les 69 variétés végétales d'orge du Tableau 2.

*Par « comparaison du ou des profil(s) obtenu(s) avec des profils de variétés connues* », on entend toute technique qui permet de déterminer, à partir d'un profil génétique obtenu selon l'invention, l'identité de la variété à l'origine du grain d'orge ayant donné ce profil. Dans un mode de réalisation préféré, le profil génétique dont l'origine est à déterminer est comparé à des profils génétiques de référence, déterminés par la méthode selon l'invention ou contenus dans une base de données et associés au nom d'une variété végétale d'orge. Ainsi, chaque variété végétale est caractérisée par un profil génétique spécifique, tel que représenté sur les Figures 2 et 3. Tout profil génétique dont l'origine est à déterminer peut être comparé aux profils de référence par tous paramètres pertinents tels que ceux cités ci-dessus, et entre autres le nombre de fragments, leur taille, leur quantité et/ou leur intensité. Dans un mode de réalisation, un profil génétique sera considéré comme identique à un profil génétique de référence lorsque ces profils ont le même nombre de fragments, et ces fragments ont exactement la même taille.

L'invention concerne également l'utilisation d'un ensemble de couples d'amorces selon l'invention pour discriminer des variétés végétales d'orge. L'utilisation d'un ensemble de couples d'amorces permet de discriminer au moins 10, au moins 20, au moins 30, au moins 40 et avantageusement au moins 50, au moins 60, au moins 70, au moins 80, au moins 90, au moins 100, au moins 200, au moins 300, au moins 400, au moins 500, au moins 1000 ou au moins 2000 variétés végétales d'orge (pures et/ou hybrides), et plus particulièrement les 69 variétés végétales d'orge listées dans le Tableau 2.

L'utilisation de l'ensemble de couples d'amorces selon l'invention s'applique aux variétés végétales selon la définition de l'article premier de la Convention internationale pour la protection des obtentions végétales (UPOV) et/ou à toute plante de l'espèce *Hordeum vulgare* qui ne répond pas à la définition d'une variété végétale selon la Convention UPOV.

Une quantité de grains d'orge peut également être hétérogène quant à sa composition. Il est ainsi souhaitable de déterminer le nombre et/ou la nature des variétés végétales présentes au sein de cette quantité. Après prélèvement d'un échantillon représentatif de la quantité initiale, les méthodes explicitées ci-dessus sont appliquées indépendamment à chaque grain d'orge constituant l'échantillon représentatif. L'identification de la variété végétale correspondant à chaque grain par les méthodes de l'invention permet ainsi :
a) de déterminer le caractère homogène (une seule variété végétale) ou hétérogène (au moins deux variétés végétales) de la quantité de grains d'orge initiale, et/ou
b) de mettre en évidence une variété végétale qui ne fait pas partie du groupe de variétés de référence choisi, et/ou
c) de mettre en évidence une plante qui ne répond pas à la définition d'une variété végétale (dans la mesure où une telle plante peut ne donner aucun fragment ou donner des fragments de taille aberrante après amplification), et/ou
d) de distinguer des variétés végétales pures de variétés végétales hybrides.

### DESCRIPTION DES FIGURES

**Figure 1** **:** Description du principe d'amplification avec un polynucléotide universel et du marquage des fragments au cours de l'amplification.

**Figure 2** **:** Exemple de profils génétiques de variétés végétales, obtenus avec un ensemble de couples d'amorces selon l'invention consistant en 5 couples d'amorces 2, 3, 7, 11 et 24 cités dans le Tableau 1 ci-dessus. De gauche à droite, on retrouve les variétés végétales suivantes :

| **Piste** | **Variété** | | **Piste** | **Variété** |
|---|---|---|---|---|
| 1 | Alexis | | 13 | Scarlett |
| 2 | Massilia | | 14 | Prestige |
| 3 | Mauritia | | 15 | Sodamm |
| 4 | Beatrix | | 16 | Esterel |
| 5 | Cervoise | | 17 | Regalia 2 |
| 6 | Belgrano | | 18 | Propice 3 |
| 7 | Chopine | | 19 | Seduction 2 |
| 8 | NFC Tipple | | 20 | Tocada 3 |
| 9 | Henley | | 21 | Sebastian |
| 10 | Cellar | | 22 | Arthurio |
| 11 | Maestria | | 23 | Carafe |
| 12 | Azurel | | | |

### EXEMPLES

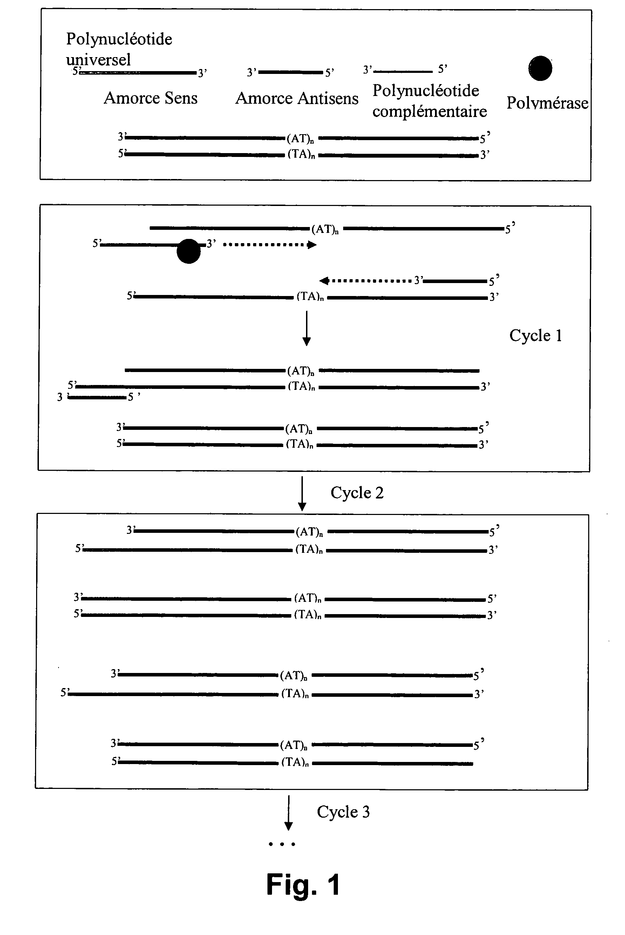

Principe : Un échantillon, après homogénéisation et réduction, est constitué de 40 grains d'une même variété de céréale ou d'une pluralité (au moins 2) variétés végétales. Les 40 grains sont broyés individuellement et subissent une lyse chimique, thermique et enzymatique. Après une brève centrifugation, l'ADN de chaque grain de l'échantillon en solution dans le surnageant est amplifié à l'aide d'au moins 5 couples d'amorces ciblant 5 loci microsatellites génétiquement non-liés et présentant un haut degré de polymorphisme. L'étape d'amplification est réalisée en présence d'un polynucléotide universel fluorescent permettant de marquer les fragments (Figure 1). Ceux-ci sont séparés par électrophorèse en gel de polyacrylamide en conditions dénaturantes et le gel est révélé en temps réel par un analyseur génétique. Les profils génétiques sont analysés à l'aide d'un logiciel de traitement d'image et comparés à une banque de données comprenant les profils préétablis. Un nom de variété est ainsi attribué à chaque grain. La composition variétale de l'échantillon de départ est déduite de ces résultats.

Etape 1 : Sélection de l'échantillon

L'échantillon pour analyse doit être homogénéisé et réduit selon la méthode BIPEA BE103D 8909, Méthode d'échantillonnage en vue d'une analyse grain par grain. Le matériel utilisé est un diviseur à rifle. La masse de la fraction du sous-échantillon à obtenir est calculée avec la formule suivante: m = (P x N x 10) / 1000, où :

P = poids de 1000 grains estimé et N = nombre de grains à prélever pour réaliser l'électrophorèse. Il faut obtenir avant la division finale une fraction ayant un poids le plus proche possible du double du poids théorique à obtenir soit, dans cet exemple, 40 g.

Un sous-échantillon de 40 grains est ensuite sélectionné dans l'échantillon avec le Numigral II. Cet appareil permet de faire un échantillonnage statistique en prélevant un grain sur 10 dans un échantillon.

### Etape 2 : Broyage des grains

Le broyage grain par grain est réalisé en plaques format 96 en Teflon®. Après ajout d'un grain et de 2 billes en tungstène ou en acier par puits, la plaque est fermée à l'aide d'une plaque de perçage en plexiglas munie de cônes en métal (un par puits). Avec le broyeur Mixer Mill® 301 (Retsch) muni d'un adaptateur à plaques, les grains d'orge sont broyés pendant 2 fois 10 minutes à une fréquence de 30 mouvements par seconde.

Etape 3 : Extraction de l'ADN

*3.1 1 Moutures et farines*

L'ADN des moutures et des farines est extrait avec le kit Nucleospin Food (Macherey Nagel), utilisé selon les informations du fournisseur. Le dosage de l'ADN est réalisé par fluorimétrie (Versafluor, Biorab) avec le kit de dosage distribué par Interchim (PicoGreen DSDNA quantification kit, Molecular Probes) et utilisé selon les informations du fournisseur.

*3.2. Grain par grain*

La technique utilisée pour extraire l'ADN repose sur une adsorption du lysat de l'échantillon brut sur un papier filtre spécialement traité pour lyser les cellules et adsorber l'ADN (papier FTA, Whatman). Après broyage à sec des échantillons, ceux ci sont additionnés de 150 µl d'eau ou de tampon de lyse (Tris HCl pH 8.0 10 mM; NaCl 150mM, EDTA 2mM, SDS 1 %) et de protéinase K (Macherey Nagel). Après une incubation de 30 minutes à 65°C, les échantillons sont additionnés de RNAse et d'α-amylase (Sigma-Aldrich), et incubés 15 minutes à température ambiante. Après une brève centrifugation, 5 µl du surnageant de chacun des échantillons est appliqué sur une carte CloneSaver (whatman), et laissé à sécher pendant 1 heure sous une hotte à flux laminaire. Une rondelle de 2 mm de diamètre est découpée et placée dans un microtube de 200 µl. Les rondelles sont lavées 2 fois avec 100 µl d'eau qualité biologie Moléculaire, 2 fois avec 100 µl d'éthanol absolu et 2 fois avec 100 µl d'eau qualité biologie moléculaire. Les tubes sont ensuite placés, ouverts, à 50°C pendant 1 h afin de sécher les rondelles de papiers.

Etape 4 : Préparation d'un ensemble de couples d'amorces

### 4.1. Sélection des couples d'amorces

Dans le tableau 3 suivant, est indiquée en minuscule la séquence du polynucléotide universel, et en majuscule la séquence propre à chaque amorce.

**Tableau 3: Séquence nucléotidique des amorces utilisées dans un ensemble de 5 couples d'amorces selon l'invention.**

| | | |
|---|---|---|
| **Couple** | **Nom** | **Séquence (de 5' vers 3')** |
| **3** | **F.HV.MS.394** | cacgacgttgtaaaacgacACCAGCAATCCAAGTTACGG |
| | **R.HV.MS.394** | TGCCTTGGTCTTGGTGTGTA |
| **24** | **F.HV.MS.420** | cacgacgttgtaaaacgacTTCTTGTGTGATACTGGCCG |
| | **R.HV.MS.420** | CTTAAAACCCCGATGAGGCT |
| **7** | **R.HV.MS.398** | cacgacgttgtaaaacgacAGCGCTTCCATCCTTGACTA |
| | **F.HV.MS.398** | TGACTGGCACTCACAACACA |
| **11** | **F.HV.MS.404** | cacgacgttgtaaaacgacTTCACCTGGCTGCTCATGTA |
| | **R.HV.MS.404** | AACTTGGAATGCTGACCCAG |
| **2** | **F.HV.MS.456** | cacgacgttgtaaaacgacAGCTTGGAGTGGAAGGGAAT |
| | **R.HV.MS.456** | CTCCACCGTTCTTCACGTTT |

*4.2. Préparation d'une composition unique contenant l'ensemble de couples d'amorces*

Chaque amorce se présente sous la forme d'une composition de concentration initiale de 1mM. Les amorces sont mélangées au sein d'une composition unique, c'est à-dire qu'un volume variant entre 1 et 10 µl selon l'amorce est ajouté dans une composition de volume final de 1 ml. La concentration finale de chaque amorce au sein de cette composition peut ainsi varier entre 1 et 10 µM, selon l'amorce. A cette solution, est également ajouté un polynucléotide complémentaire du polynucléotide universel lié en 5' des amorces sens, pour permettre la détection des fragments d'ADN amplifiés, dont la concentration finale varie entre 10 et 20 µM, et est avantageusement 20 µM.

Etape 5 : PCR multiplex

*5.1 Préparation des réactifs d'amplification*

Les différents réactifs permettant l'amplification multiplex sont mélangés dans les proportions indiquées dans le Tableau 4 suivant. Les volumes s'entendent pour l'amplification de l'ADN d'un grain d'orge. La concentration de l'ADN d'orge est indiquée en génome (Go), 1 Go étant égal à une concentration d'ADN de 17 pg/µl.

**Tableau 4: Préparation du mélange réactionnel pour une réaction d'amplification multiplex dans 10 µl final.**

| **Mix simplex pour identification** | | **Conc finale** |
|---|---|---|
| **Nombre de réactions** | **1** | |
| **ADN (1000 Génomes/µl)** | 1 µl | 1000 Go |
| **Mélange Amorces/Sonde** | 0,5 µl | 3,7 µM |
| **Master Mix (Multiplex Qiagen)** | 5 µl | 1X |
| **Eau qsp 10** | 3,5 µl | |
| **Total** | 10 µl | |

A ces réactifs d'amplification, est ajouté le mélange amorces/sonde préparé selon le point 4.2 ci-dessus de manière à obtenir une concentration finale en amorce au sein du volume réactionnel variant entre 25 nM et 1 µM.

Le tableau 5 suivant indique les concentrations optimales d'amorces et du polynucléotide complémentaire (Poly. compl.) au sein du volume réactionnel ([réaction]) dans la réalisation du procédé de l'invention, et plus particulièrement pour les conditions de préparation indiquées ci-dessus.

**Tableau 5 : Concentration finale optimale des amorces, au sein d'un mélange réactionnel d'amplification, de l'ensemble défini au tableau 3.**

| **Amorces** | **[réaction]** |
|---|---|
| **F.HV.MS.394** | 250 nM |
| **R.HV.MS.394** | 250 nM |
| **F.HV.MS.420** | 250 nM |
| **R.HV.MS.420** | 250 nM |
| **R.HV.MS.398** | 100 nM |
| **F.HV.MS.398** | 100 nM |
| **F.HV.MS.404** | 250 nM |
| **R.HV.MS.404** | 250 nM |
| **F.HV.MS.456** | 250 nM |
| **R.HV.MS.456** | 250 nM |
| **Poly. compl** | 1 µM |

*5.2. Programme PCR*

10 µl de mélange réactionnel de PCR sont ajoutés directement dans chacun des tubes. Les tubes sont ensuite scellés et le milieu réactionnel est incubé selon le programme d'amplification (Tableau 6) préconisé par le fournisseur de l'enzyme. L'appareil utilisé est soit le thermocycleur Master Cycle (Eppendorf) soit GeneAmp 9700 (Applied Biosystem).

**Tableau 6: Programme d'amplification d'un ensemble de couples d'amorces selon l'invention. Tous les ensembles de couples d'amorces spécifiques amplifient à une température de 60°C dans les conditions décrites.**

| **Etape** | **Durée** | **Température** | |
|---|---|---|---|
| **1** | 15 min | 95°C | |
| **2** | 1 min | 95°C | |
| **3** | 1 min 30 sec | **60°C** | 40 cycles |
| **4** | 1 min | 72°C | |
| **5** | 2 min | 72°C | |
| **6** | 1 min | 20°C | |

Etape 6: Détection de l'ensemble de fragments d'ADN amplifiés

*6.1. Séparation des fragments*

Après l'amplification, les fragments sont additionnés de 10% de solution de dépôt (Formamide 80%, EDTA 10 mM, Bleu de bromophénol, 2 mg/ml) et dénaturés par chauffage à 95°C, 2 minutes. La séparation en fonction de la taille est réalisée par électrophorèse en gel de polyacrylamide dénaturant (USB RapidGel 6% Ultrapure, Amersham) (Dimensions du gel 25 cm X 15 cm X 0,2 mm) (paramètres de migration: 1500V, 40 mA, 40W, 50°C). L'appareil utilisé est un analyseur génétique LiCor NEN2 Global Controler System.

*6.2 Visualisation des fragments*

Marquage IRD 800 (système à 3 amorces) : un polynucléotide universel est inséré en 5' de chacune des amorces sens. L'amplification est réalisée en présence d'un troisième polynucléotide, complémentaire du polynucléotide universel. Ce troisième polynucléotide est modifié en 5' par l'ajout d'une molécule fluorescente émettant dans l'infrarouge à 800 nm. Lors des cycles d'amplification, la molécule fluorescente, par incorporation aux fragments d'amplification, sert à marquer ces fragments. Le principe du marquage est schématisé sur la Figure 1.

Durant la migration des fragments d'ADN amplifiés, le laser Infrarouge de l'analyseur génétique balaie le bas du gel à travers une fenêtre de 3 x 15 cm et collecte les informations de fluorescence qui sont numérisée sous le format TIFF.

Etape 7 : Comparaison du ou des profils avec des profils de référence

L'image du gel est analysée à l'aide du logiciel Bionumerix, qui compare les profils obtenus à une banque de profils préétablis sur les variétés végétales d'orge pures connues.

### RESULTATS

**A. Couples d'amorces monomorphes, faiblement polymorphes ou ne conduisant pas à une amplification efficace dans les conditions multiplex retenues**

Afin de déterminer les couples d'amorces appropriés pour la réalisation d'un ensemble de couples d'amorces utilisable au sein d'une réaction multiplex et permettant de discriminer un grand nombre de variétés végétales, le caractère polymorphes des loci ont été testés. De plus, certains couples d'amorces, bien qu'ils présentent un caractère polymorphe, n'ont pas été considérés dans la mesure où leur utilisation au sein d'ensemble de couples d'amorces selon l'invention ne permettait pas une amplification efficace, dans un même volume réactionnel, avec tous les couples d'amorces de cet ensemble. Ceci s'explique notamment par la température d'hybridation propre à chaque couple, les éventuels phénomènes de compétition entre amorces et l'hybridation des amorces entre elles ou avec un produit d'amplification et l'absence d'amplification lorsque certaines amorces sont utilisées dans des conditions multiplex (stoechiométrie).

Les marqueurs listés dans le Tableau 7 se sont révélés monomorphes lorsqu'une amplification a été réalisée sur des variétés végétales d'orge.

**Tableau 7 : Exemple de couples d'amorces non utilisables pour former un ensemble de couples d'amorces selon l'invention.**

| | |
|---|---|
| **FHVMS401** | TGCAGATGATGCCTTCTGTC |
| **RHVMS401** | TGCTGCTCAACAACCATAGC |
| **FHVMS403** | CGGACGAGGTTTACTCCAAA |
| **RHVMS403** | GCTCTACGATGCATCCCATT |
| **FHVMS409** | GATAACATCCCGGGGCTAAT |
| **RHVMS409** | GGATGACATGGCTCTGGTTT |
| **FHVMS421** | TGTGACTGCTTCTGCAGCTT |
| **RHVMS421** | TGTATTCGCCCACACCAGTA |
| **FHVMS428** | ACAAAATGAGGTCCTCGTGC |
| **RHVMS428** | TCCACAAGTTCTTCCAGGCT |
| **FHVMS432** | GAGGGGCACAAGGAAGAAAT |
| **RHVMS432** | TGTGGCACATCCAGGAATAG |
| **FHVMS433** | TTGCCTTAACCCTGGTCATC |
| **RHVMS433** | GGAATCGTGTCACGTCCTTT |
| **FHVMS434** | GGTGAGGGCAAAAGGGTAAT |
| **RHVMS434** | TCGTCATGGCAGAAGTGTGT |

**B. Exemple de profils obtenus avec des ensembles de couples d'amorces selon l'invention**

Le tableau 8 suivant liste le nombre d'allèles obtenus avec les ensembles de couples d'amorces cités ci-dessus, ainsi que la taille respective de ces allèles, et le nombre de profils théoriques que l'on peut obtenir en considérant que le procédé de l'invention est appliquée à des variétés pures, c'est-à-dire présentant deux allèles de même taille.

**Tableau 8 : Nombre de profils génétiques théoriques avec l'ensemble de couples d'amorces défini au tableau 3.**

| **Couple** | **Longueur moyenne des fragments** | **Nombre d'allèles** | **Nombre de profils théoriques** |
|---|---|---|---|
| 2 | 122 pb | 3 | |
| 3 | 124 pb | 3 | |
| 7 | 139 pb | 3 | 324 |
| 11 | 179 pb | 3 | |
| 24 | 249 pb | 4 | |

Le nombre de profils génétiques théoriques, calculé en fonction du nombre d'allèles par couple d'amorces, est d'environ 324 pour une variété pure. Ceci reflète la possibilité de discriminer un grand nombre de variétés végétales, si ce n'est toutes les variétés végétales existantes, et à tout le moins les 69 variétés végétales identifiées dans le tableau 2. Il est bien entendu que le nombre de profils génétiques théoriques est bien plus grand lorsque l'amplification est réalisée sur des variétés hybrides, dans la mesure où une variété hybride peut présenter deux allèles de taille différente pour un même locus.

Un autre exemple du nombre de profils génétiques théoriques calculé pour un autre ensemble d'amorces selon l'invention est résumé dans le tableau 9 ci-dessous :

**Tableau 9 : Nombre de profils génétiques théoriques avec l'ensemble de couples d'amorces 2, 7, 11, 22, 24 et 33.**

| **Couple** | **Longueur moyenne des fragments** | **Nombre d'allèles** | **Nombre de profils théoriques** |
|---|---|---|---|
| 2 | 122 pb | 3 | 1620 |
| 7 | 139 pb | 3 | |
| 11 | 179 pb | 3 | |
| 22 | 242 pb | 5 | |
| 24 | 249 pb | 4 | |
| 33 | 268 pb | 3 | |

La Figure 2 représente un exemple de gel d'électrophorèse obtenu avec un ensemble de 5 couples d'amorces selon l'invention, à savoir les couples 2, 3, 7, 11 et 24.

Le gel comprend 23 pistes correspondant à 23 variétés végétales d'orge. Avec l'ensemble de couples d'amorces utilisé, toutes les variétés végétales sur lesquelles a été réalisée l'amplification présentent un profil génétique spécifique, c'est-à-dire qu'aucune des variétés végétales ne présente un profil identique à une autre variété utilisée. Ces résultats confirment d'une part que, de par la sélection de couples d'amorces appropriés, il est possible de discriminer un grand nombre de variétés végétales avec un faible nombre de couples d'amorces (au moins 5 couples), et d'autre part que l'amplification d'au moins 5 couples dans des conditions multiplex (au sein d'un même volume réactionnel) permet d'obtenir, dans des variétés pures, un fragment d'amplification unique par locus amplifié. Ceci valide donc le caractère sûr, rapide et bon marché de la méthode par rapport aux méthodes de discrimination conventionnelles.

## Revendications

1. Ensemble de couples d'amorces, dans lequel au moins 5 couples d'amorces, constitués d'une amorce sens et d'une amorce antisens, sont choisis parmi les couples suivants :
| **Couple** | **Amorce sens** | **Seq ID** | **Amorce antisens** | **Seq ID** |
|---|---|---|---|---|
| **1** | AAGACACACACACACACGCA | 1 | CGAACTCCATGATGAATCCC | 2 |
| **2** | AGCTTGGAGTGGAAGGGAAT | 3 | CTCCACCGTTCTTCACGTTT | 4 |
| **3** | ACCAGCAATCCAAGTTACGG | 5 | TGCCTTGGTCTTGGTGTGTA | 6 |
| **4** | CTCGATATGCATGCTTGGTG | 7 | CCCGGCTACCAAATGAGTAA | 8 |
| **5** | CAGAGAACCAAGCCAAAACC | 9 | CTATGTTTGGTCCCCTTGGA | 10 |
| **6** | TCCGTACATGGGATGCAGTA | 11 | CACGTTTAACAGGACCGACA | 12 |
| **7** | AGCGCTTCCATCCTTGACTA | 13 | TGACTGGCACTCACAACACA | 14 |
| **8** | TGTCTCCCATCAAGGTTTCC | 15 | TCCTCTTCTTTGCGCTCTTC | 16 |
| **9** | AGCTTGGATGTCTGGCAGTT | 17 | AGACCAAATTCACAGGCCAC | 18 |
| **10** | GCTAAACCCATCAGTGGCAT | 19 | CAAAATTGCTCTCCTCAGGG | 20 |
| **11** | TTCACCTGGCTGCTCATGTA | 21 | AACTTGGAATGCTGACCCAG | 22 |
| **12** | TCGAGCAGGTCAAGAAGGAT | 23 | CACGGAGACATGCATAAACG | 24 |
| **13** | TTTTCTGGTGTGTGTGAGGC | 25 | TGTCAAGAACCTGCTGTTGC | 26 |
| **14** | GGAGGAAAATAGGCACGGAT | 27 | GGCATCTGCTCTTGATGACA | 28 |
| **15** | GATTCGCTGATCGACCACTT | 29 | ATCGATACAACAGTGTGCCG | 30 |
| **16** | GATTCGCTGATCGACCACTT | 31 | ATCGATACAACAGTGTGCCG | 32 |
| **17** | GGTTGCCAGAAGGATGTGAT | 33 | TCGCATAGGCAGATACACCA | 34 |
| **18** | ATGACCAGAAAACGCCTGTC | 35 | GGATTCTGCACACACGAGAA | 36 |
| **19** | TGTCTTTAGGAACGGAGGGA | 37 | CAACACCCAGATGACGTTTG | 38 |
| **20** | CCTGTTCTCCATGTTTGGCT | 39 | ATCAGTCCGGAAAGACATGC | 40 |
| **21** | GATTCGCTGATCGACCACTT | 41 | ATCGATACAACAGTGTGCCG | 42 |
| **22** | TGCCATCTCAAGAACACGAG | 43 | AAGAGAACTTCCGCTCCACA | 44 |
| **23** | CAACTCATTCGTCGGTGATG | 45 | GGGGTTGGTGACAACTCAAT | 46 |
| **24** | TTCTTGTGTGATACTGGCCG | 47 | CTTAAAACCCCGATGAGGCT | 48 |
| **25** | TGAAGGAGATCAAGAACGGG | 49 | ATCAGTCCGGAAAGACATGC | 50 |
| **26** | TGAAGGAGATCAAGAACGGC | 51 | ATCAGTCCGGAAAGACATGC | 52 |
| **27** | GACATGTTGGAAGGTGGCTT | 53 | CTGTCAGCTCGGACAACAAA | 54 |
| **28** | TGCTATCACACCAATCAGGG | 55 | TGGACATAGTCCTGGCAACA | 56 |
| **29** | ACCATCCCCACGTAGGATTT | 57 | TTTTTCCTCCTCCTCGTGTG | 58 |
| **30** | CACACCGCACACAACATACA | 59 | TTCGTTGCATAGTGGACAGC | 60 |
| **31** | CAGTTGGCTTCTACCCCAAA | 61 | GCTACGACCCACAACAACAA | 62 |
| **32** | AGTGTGCTTGAGGCCAAGAT | 63 | GAGGATTCGCTCAAAGTTGC | 64 |
| **33** | TCTGCACAGCTGTTTGGTTC | 65 | CAAGCAGATGCAACTACACCA | 66 |

2. Ensemble de couples d'amorces selon la revendication 1 qui consiste en 5 couples d'amorces.

3. Ensemble de couples d'amorces selon l'une quelconque des revendications 1 à 2, qui permet d'amplifier au moins 3 allèles, et de préférence au moins 5 allèles, par locus amplifié par lesdits couples d'amorces dans l'ensemble testé des variétés végétales d'orge.

4. Ensemble de couples d'amorces selon l'une quelconque des revendications 1 à 3, dans lequel les loci sont non-liés génétiquement.

5. Ensemble de couples d'amorces selon l'une quelconque des revendications 1 à 4, permettant d'obtenir un ensemble de fragments d'ADN spécifique d'une variété végétale d'orge parmi des variétés végétales d'orge connues constituant un groupe de référence.

6. Ensemble de couples d'amorces selon l'une quelconque des revendications 1 à 5 permettant d'obtenir un ensemble de fragments d'ADN spécifique d'une variété végétale d'orge parmi au moins 50 variétés végétales d'orge connues constituant un groupe de référence.

7. Ensemble de couples d'amorces selon l'une quelconque des revendications 1 à 6, choisi parmi les combinaisons de couples d'amorces suivantes :
a) 2, 3, 7, 11 et 24,
b) 2, 10, 16, 17 et 31,
c) 1, 11, 16, 18 et 32, et
d) 2, 7, 11, 22, 24 et 33.

8. Ensemble de couples d'amorces selon l'une quelconque des revendications 1 à 7, dans lequel au moins une amorce est un variant fonctionnel d'une amorce du tableau 1.

9. Ensemble de couples d'amorces selon l'une quelconque des revendications 1 à 8 dans lequel chaque amorce sens possède, attaché en 5', un polynucléotide universel.

10. Ensemble de couples d'amorces selon la revendication 9, dans lequel le polynucléotide universel possède la séquence CCAGGACGTTGTAAAACGAC (Seq ID N° 67).

11. Ensemble de fragments d'ADN tel qu'obtenu par amplification à partir de l'ADN d'orge d'une variété donnée, au moyen d'un ensemble de couples d'amorces, dans lequel au moins 5 couples d'amorces sont choisis parmi les couples désignés 1 à 33 de l'une quelconque des revendications 1 à 10, ledit ensemble de fragments d'ADN étant spécifique d'une variété végétale d'orge.

12. Ensemble de fragments d'ADN selon la revendication 11, ledit ensemble de fragments d'ADN étant spécifique d'une variété végétale d'orge parmi au moins 50 variétés végétales d'orge connues constituant un groupe de référence.

13. Ensemble de fragments d'ADN selon l'une quelconque des revendications 11 à 12, lesdits fragments étant amplifiés par un ensemble de couples d'amorces choisi parmi : a) 2, 3, 7, 11 et 24, b) 2, 10, 16, 17 et 31, c) 1, 11, 16, 18 et 32, et d) 2, 7, 11, 22, 24 et 33.

14. Kit d'obtention d'un ensemble de fragments d'ADN spécifique d'une variété végétale d'orge comprenant :
a. un ensemble de couples d'amorces, dans lequel au moins 5 couples d'amorces sont choisis parmi les couples 1 à 33 de l'une quelconque des revendications 1 à 10, et
b. au moins une solution servant de marqueur de taille pour lesdits fragments.

15. Kit selon la revendication 14, dans lequel une solution servant de marqueur de taille est un ensemble de fragments amplifiés par les couples d'amorces (1 à 33) spécifique d'une variété végétale d'orge, et préférentiellement d'au moins une variété végétale choisie parmi Alexis, Massilia, Mauritia, Beatrix, Cervoise, Belgrano, Chopine, NFC Tipple, Helney Azurel et Sodamm.

16. Kit selon l'une quelconque des revendications 14 à 15, dans lequel l'ensemble de couples d'amorces est choisi parmi : a) 2, 3, 7, 11 et 24, b) 2, 10, 16, 17 et 31, c) 1, 11, 16, 18 et 32, et d) 2, 7, 11, 22, 24 et 33.

17. Kit selon l'une quelconque des revendications 14 à 16, dans lequel les au moins 5 couples d'amorces sont présentés sous forme d'une composition unique.

18. Kit selon l'une quelconque des revendications 14 à 17, comprenant en outre au moins une solution d'ADN d'une variété végétale d'orge connue.

19. Kit selon l'une quelconque des revendications 14 à 18, comprenant en outre les réactifs nécessaires à l'amplification : une polymérase et des désoxyribonucléotides tri-phosphates ou dNTP (dGTP, dATP, dTTP et dCTP).

20. Kit selon l'une quelconque des revendications 14 à 19, comprenant en outre un polynucléotide complémentaire dudit polynucléotide universel.

21. Kit selon la revendication 20, dans lequel le polynucléotide complémentaire est marqué.

22. Méthode d'obtention d'un profil génétique spécifique d'une variété végétale d'orge comprenant :
a. l'amplification simultanée d'au moins 5 loci d'une solution d'acides nucléiques obtenue à partir d'un produit dérivé d'orge, au sein d'un unique mélange réactionnel, par un ensemble de couples d'amorces selon l'une quelconque des revendications 1 to 10, conduisant à l'obtention d'un ensemble de fragments d'ADN amplifiés, et
b. la détection de l'ensemble formé par lesdits fragments d'ADN amplifiés et l'obtention d'un profil génétique.

23. Méthode d'obtention d'un profil génétique selon la revendication 22, dans laquelle la solution d'acides nucléiques est obtenue à partir d'un grain d'orge, du malt d'orge, du broyat de grains ou de malt d'orge, de la farine d'orge, de la bière, tout produit de malterie/brasserie, ou tout produit contenant de l'orge, du malt d'orge ou du malt spécial.

24. Méthode d'obtention d'un profil génétique selon l'une quelconque des revendications 22 à 23, dans laquelle l'amplification est réalisée par réaction de polymérisation en chaîne (PCR).

25. Méthode d'obtention d'un profil génétique selon l'une quelconque des revendications 22 à 24, dans laquelle la concentration finale de chaque amorce permettant la réaction d'amplification est comprise entre 10 nM et 1 µM.

26. Méthode d'obtention d'un profil génétique selon l'une quelconque des revendications 22 à 25, dans laquelle la concentration finale d'ADN sur lequel est réalisée l'amplification est comprise entre 10 et 20 ng/µl.

27. Méthode d'obtention d'un profil génétique selon l'une quelconque des revendications 22 à 26 dans laquelle la détection desdits fragments d'ADN amplifiés requiert leur séparation puis leur visualisation.

28. Méthode d'obtention d'un profil génétique selon la revendication 27, dans laquelle lesdits fragments d'ADN amplifiés sont séparés selon un critère de taille.

29. Méthode d'obtention d'un profil génétique selon la revendication 28, dans laquelle lesdits fragments d'ADN amplifiés sont séparés par migration électrophorétique.

30. Méthode d'obtention d'un profil génétique selon l'une quelconque des revendications 22 à 29 dans laquelle la détection ou la visualisation des fragments d'amplification est réalisée par fluorescence.

31. Méthode de discrimination de variétés végétales de céréales comprenant une méthode d'obtention d'un profil génétique selon l'une quelconque des revendications 22 à 30, et une étape c. de comparaison du ou des profil(s) obtenu(s) avec des profils de variétés connues.

32. Méthode de discrimination de variétés végétales de céréales selon la revendication 31 permettant de discriminer au moins 50 variétés végétales d'orge.
